# EUROPEAN PATENT APPLICATION

(11) **EP 2 364 725 A2**
(43) Date of publication of application: **14.09.2011**
(21) Application number: 10183385.3
(22) Date of filing: 23.06.2004
(51) Int. Cl.: A61K 39/095, A61P 31/00

(54) **Immunization method against neisseria meningitidis serogroups a and c**

(30) Priority: 23.06.2003 US 480925 P
(62) Divisional of application: 04755949.7
(71) Applicant: Sanofi Pasteur Inc., Swiftwater, PA 18370 (US)
(72) Inventor: Ryall, Robert, Swiftwater, Pennsylvania 18370 (US)
(74) Representative: Sahans, Sarah Anne

(57) **Abstract**

The present invention describes methods of immunizing a patient with a combined vaccine that offers protection against meningococcal disease caused by pathogenic bacteria *Neisseria meningitidis serogroups* A and C. The vaccine comprises at least two distinct polysaccharide-protein conjugates that are formulated as a single dose of vaccine. The purified capsular polysaccharides of *Neisseria meningitidis serogroups* A and C are chemically activated and selectively attached to a carrier protein by means of a covalent chemical bond, forming polysaccharide-protein conjugates capable of eliciting long-lasting immunity to a variety of *N. meningitidis* strains in infants.

## Description

The present application claims priority to U.S. provisional application No.: 60/480,925 filed on June 23, 2003, the entire disclosure of which is herein incorporated by reference in its entirety.

### Field of the Invention

The present invention relates to the field of medicine generally, and more specifically to microbiology, immunology, vaccines and the prevention of infection by a bacterial pathogen by immunization.

### Background of the Invention

*Neisseria meningitidis* is a leading cause of bacterial meningitis and sepsis throughout the world. The incidence of endemic meningococcal disease during the last thirty years ranges from 1 to 5 per 100,000 in the developed world, and from 10 to 25 per 100,000 in developing countries (Reido, F.X., *et al.* 1995). During epidemics the incidence of meningococcal disease approaches 1000 per 1000,000. There are approximately 2,600 cases of bacterial meningitis per year in the United States, and on average 330,000 cases in developing countries. The case fatality rate ranges between 10 and 20%.

Pathogenic meningococci are enveloped by a polysaccharide capsule that is attached to the outer membrane surface of the organism. Thirteen different serogroups of meningococci have been identified on the basis of the immunological specificity of the capsular polysaccharide (Frasch, C.E., *et al.* 1985). Of these thirteen serogroups, five cause the majority of meningococcal disease; these include serogroups A, B, C, W-135, and Y. Serogroup A is responsible for most epidemic disease. Serogroups B, C, and Y cause the majority of endemic disease and localized outbreaks.

The human naso-oropharyngeal mucosa is the only known natural reservoir of *Neisseria meningitidis.* Colonization takes place both at the exterior surface of the mucosal cell and the subepithelial tissue of the nasopharynx. Carriage of meningococci can last for months. Spreading of meningococci occurs by direct contact or via air droplets. Meningococci become invasive by passing through the mucosal epithelium via phagocytic vacuoles as a result of endocytosis. Host defense of invasive meningococci is dependent upon complement-mediated bacteriolysis. The serum antibodies that are responsible for complement-mediated bacteriolysis are directed in large part against the outer capsular polysaccharide.

Vaccines based on meningococcal polysaccharide have been described which elicit an immune response against the capsular polysaccharide. These antibodies are capable of complement-mediated bacteriolysis of the serogroup specific meningococci. The meningococcal polysaccharide vaccines are shown to be efficacious in children and adults (Peltola, H., *et al.* 1977 and Artenstein, M.S., *et al.* 1970), but the efficacy is limited in infants and young children (Reingold, A.L., *et al.* 1985). Subsequent doses of the polysaccharide in younger populations elicited a weak or no booster response (Goldschneider, I., *et al.* 1973 and Gold, R., *et al.* 1977). The duration of protection elicited by the meningococcal polysaccharide vaccines is not long lasting, and has been estimated to be between 3 to 5 years in adults and children above four years of age (Brandt, B., *et al.* 1975, Käyhty, H., *et al.* 1980, and Ceesay, S. J., *et al.* 1993). For children from one to four years old the duration of protection is less than three years (Reingold, A.L., *et al.* 1985).

Polysaccharides are incapable of binding to the major histocompatibility complex molecules, a prerequisite for antigen presentation to and stimulation of T-helper lymphocytes, i.e., they are T-cell independent antigens. Polysaccharides are able to stimulate B lymphocytes for antibody production without the help of T-helper lymphocytes. As a result of the T-independent stimulation of the B lymphocytes, there is a lack of memory induction following immunization by these antigens. The polysaccharide antigens are capable of eliciting very effective T-independent responses in adults, but these T-independent responses are weak in the immature immune system of infants and young children.

T-independent polysaccharide antigens can be converted to T-dependent antigens by covalent attachment of the polysaccharides to protein molecules ("carriers" or "carrier proteins"). B cells that bind the polysaccharide component of the conjugate vaccine can be activated by helper T cells specific for peptides that are a part of the conjugated carrier protein. The T-helper response to the carrier protein serves to augment the antibody production to the polysaccharide. Conjugation to a carrier protein has not always resulted in a vaccine capable of inducing memory against the polysaccharide.

MacLennan *et al.* describe a study of a meningococcal A/C adjuvanted conjugate vaccine given to infants, less than six months old. MacLennan, J. et al., J. Infect.Dis. 2001;183:97-104. The conjugate vaccine contained 11 µg of each polysaccharide and 49 µg of CRM197 adjuvanted with 1 mg of aluminum hydroxide. The children are boosted with either a mengococcal A/C polysaccharide vaccine containing 50 µg of each polysaccharide or the conjugate when the children are between 18 and 24 months, and revaccinated at about 5 years of age with a single meningococcal A/C vaccine containing 10 µg of each polysaccharide. Blood samples are drawn at pre-vaccination and ten days post-vaccination. The authors noted that prevaccination Group A antibody concentrations are high in all groups, and concluded that they did not believe that immunologic memory to the group A component of this vaccine is conclusively proven.

The serogroup B polysaccharide has been shown to be poorly to non-immunogenic in the human population (Wyle, F.A., *et al.* 1972). Chemical attachment of this serogroup polysaccharide to proteins has not significantly altered the immune response in laboratory animals (Jennings, H. J., *et al.* 1981). The reason for the lack of immune response to this serogroup polysaccharide is thought to arise from structural similarities between the serogroup B polysaccharide and polysialylated host glycoproteins, such as the neural cell adhesion molecules.

A meningococcal conjugate vaccine based on serogroup C polysaccharide has been described. This monovalent vaccine elicits a strong functional antibody response to the capsular polysaccharide present on strains of *N. meningitidis* corresponding to serogroup C. Such a vaccine is only capable of protecting against disease caused by serogroup C bacteria.

USP 5,425,946 describes an immunogenic conjugate comprising a modified group C meningococcal polysaccharide (GCMP) coupled to a carrier molecule. The GCMP is modified by O-deacetylation to a varying extent. The patent describes selectively removing the O-acetyl groups on positions 7 and/or 8 of the sialyl moieties in the group C polysaccharide from OAc+ strains are to a varying extent from the meningococcal group C polysaccharide by treatment with an appropriate reagent.

Methods for making polysaccharide-protein conjugates using an adipic dihydrazide spacer is described by Schneerson, R., et al, Preparation, Characterization and Immunogenicity of Haemophilus Influenzae Type b Polysaccharide-Protein Conjugates, J. Exp. Med., 1952, 361-476 (1980), and in U.S. Pat. No. 4,644,059 to Lance K. Gordon. Other linker methods, such as a binary spacer technology as described by Marburg, S., et al, "Biomolecular Chemistry of Macromolecules: Synthesis of Bacterial Polysaccharide Conjugates with Neisseria meningitidus Membrane Protein", J. Am. Chem. Soc., 108, 5282-5287 (1986) and a reducing ends methodology, as referred to by Anderson in U.S. Pat. No. 4,673,574 are known.

Existing vaccines based on meningococcal polysaccharide are of limited use in young children and do not provide long-lasting protection in adults. The only meningococcal vaccine which as been shown to be capable of eliciting long-lasting protection in all groups, including children, at risk for meningococcal infection is based on a polysaccharide from a single serogroup of *N*. *meningitidis* and provides no protection against infection by other serogroups. Thus, a need exists for a meningococcal conjugate vaccine capable of conferring broad, long-lived protection against meningococcal disease in children and adults at risk for meningococcal infection. The multivalent meningococcal polysaccharides of the present invention solve this need by providing vaccine formulations in which immunogenic polysaccharides from the major pathogenic serogroups *of N. meningitidis* have been converted to T-dependent antigens through conjugations to carrier proteins.

### SUMMARY OF THE INVENTION

The present invention provides a method for prevention of diseases caused by pathogenic *Neisseria meningitidis* serogroups A and C by administration of a composition comprising aluminum-free meningococcal polysaccharide-protein conjugates.

The present invention provides a method of inducing an immunological response to capsular polysaccharide serogroups A and C of *N. meningitidis* by administering an immunologically effective amount of the immunological composition to a human. The immunological composition is a multivalent meningococcal vaccine comprising at least two distinct protein-polysaccharide conjugates, one conjugate comprising a capsular polysaccharide of serogroup A conjugated, either directly or by a linker, to a carrier protein, and a second conjugate comprising a capsular polysaccharide of serogroup C conjugated, either directly or by a linker, to a carrier protein. The immunological composition is aluminum-free. The immunological composition may contain other compounds, such as aluminum-free adjuvants, or preservatives.

All patents, patent applications, and other publications recited herein are hereby incorporated by reference in their entirety.

### DETAILED DESCRIPTION OF THE INVENTION

The present invention provides a method of inducing an immunological response to capsular polysaccharides of serogroups A and C *of N. meningitidis* by administering to a human an aluminum-free immunologically effective amount of the immunological composition comprising capsular polysaccharides of serogroups A and C each conjugated to a carrier protein. The capsular polysaccharides of serogroups A and C are preferably individually conjugated to a carrier protein. Conjugation may be a direct chemical linkage between the polysaccharide and the carrier protein, or an indirect linkage whereby the polysaccharide and carrier protein are each chemically via a chemical linker molecule. The polysaccharide may be first covalently attached to the linker molecule, then the carrier protein covalently attached to the linker molecule. Alternatively, the carrier protein may be first covalently attached to the linker molecule, then the polysaccharide attached to the linker molecule. The immunological composition may contain other compounds, such as aluminum-free adjuvants, or preservatives.

Methods to prepare capsular polysaccharides *of N. meningitidis* serogroups A and C are well known in the art, as vaccines containing *N. meningitidis* polysaccharides have been licensed for many years. For example, methods for obtaining capsular polysaccharides from serogroup A of N. meningitidis are described in Moreau U.S. Patent 6,045,805, using a method described in Gotschlich et al., Prog. Immunobiol. Standard. (1972) 5: 485. USP 6,045,805 describes preparing an oligosaccharide from a larger, native polysaccharide by depolymerizing the polysaccharide and eluting the smaller oligosaccharide from a chromatography column. The oligosaccharide may be isolated using a number of conventional techniques, for example, by precipitation using an appropriate precipitating agent such as acetone or alcohol, by filtration on a membrane having an appropriate separation threshold, by exclusion-diffusion or by ion-exchange chromatography. Subsequently, oligosaccharide fractions containing molecules having an elution constant equal to, or in the vicinity of, the mean elution constant may be obtained.

The polysaccharide according to the invention may be coupled, via covalent bonding, with a compound of peptide or protein nature or with another organic polymer such as for example polyacrlate in order to form a conjugate capable of promoting the immunogenicity of the polysaccharide especially in a mammal. It is preferred that the polysaccharide is conjugated to a bacterial protein, more preferably, a bacterial toxin, the corresponding anatoxin or a subunit of a multimeric toxin as well as a membrane protein, a subunit of a multimeric membrane protein or a cytoplasmic protein. Preferred toxins include, pertussis toxin, cholera toxin, tetanus toxin and diphtheria toxin. These proteins can be extracted from the original bacteria or alternatively can be made recombinantly.

Chemical methods for preparing polysaccharide-protein conjugates are well known. For example, a functional group may be created on the oligosaccharide which is capable of reacting with a functional group of the carrier protein. A bifunctional coupling agent may also be reacted with the oligosaccharide and then with a carrier protein, or vice versa. W. E. Dick and M. Beurret in Conjugates Vaccines, J. M. Cruse, R. E. Lewis Jr Eds, Contrib. Microbiol. Immunol. Basel, Karger (1989) 10 : 48 provides a review of these various coupling methods. Furthermore, the oxidation-reduction fragmentation process introduces reducing groups, especially into the oligosaccharide derived from a polysaccharide of N. *meningitidis* group A.

In a preferred embodiment, these meningococcal serogroup conjugates are prepared by separate processes and formulated into a single dosage formulation. For example, capsular polysaccharides from serogroups A and C *of N. meningitidis* are separately purified.

In a preferred embodiment of the present invention, the purified A and C polysaccharides are separately depolymerized and separately activated prior to conjugation to a carrier protein. Preferably, the capsular polysaccharides of serogroups A and C of *N. meningitidis* are partially depolymerized separately using mild oxidative conditions.

The depolymerization or partial depolymerization of the polysaccahrides may then be followed by an activation step. By "activation" is meant chemical treatment of the polysaccharide to provide chemical groups capable of reacting with the carrier protein. A preferred activation method involves treatment with adipic acid dihyrazide in physiological saline at pH 5.0±0.1 for approximately two hours at 15 to 30°C. One process for activation is described in U.S. Patent 5,965,714.

Once activated, the capsular polysaccharides may then be conjugated to one or more carrier proteins. In a preferred embodiment of the present invention, each A and C capsular polysaccharide is separately conjugated to a single carrier protein, more preferably, each is conjugated to the same carrier protein.

Carrier proteins may include inactivated bacterial toxins such as diphtheria toxoid, CRM¹⁹⁷, tetanus toxoid, pertussis toxoid, *E. coli* LT, *E. coli* ST, and exotoxin A from *Pseudomonas aeruginosa.* Bacterial outer membrane proteins such as, outer membrane complex c (OMPC), porins, transferrin binding proteins, pneumolysis, pneumococcal surface protein A (PspA), or pneumococcal adhesin protein (PsaA), could also be used. Other proteins, such as ovalbumin, keyhole limpit hemocyanin (KLH), bovine serum albumin (BSA) or purified protein derivative of tuberculin (PPD) may also be used as carrier proteins. Carrier proteins are preferably proteins that are non-toxic and non-reactogenic and obtainable in sufficient amount and purity. Carrier proteins should be amenable to standard conjugation procedures. In a preferred embodiment of the present invention diphtheria toxin purified from cultures of *Corynebacteria diphtheriae* and chemically detoxified using formaldehyde is used as the carrier protein.

After conjugation of the capsular polysaccharide to the carrier protein, the polysaccharide-protein conjugates may be purified (enriched with respect to the amount of polysaccharide-protein conjugate) by a variety of techniques. One goal of the purification step is to remove the unbound polysaccharide from the polysaccharide-protein conjugate. One method for purification, involving ultrafiltration in the presence of ammonium sulfate, is described in U.S. Patent 6,146,902. Alternatively, conjugates can be purified away from unreacted protein and polysaccharide by any number of standard techniques including, *inter alia,* size exclusion chromatography, density gradient centrifugation, hydrophobic interaction chromatography or ammonium sulfate fractionation. See, e.g., P.W. Anderson, et al. (1986). J. Immunol. 137: 1181-1186. See also H. J. Jennings and C. Lugowski (1981) J. Immunol. 127:1011-1018.

After conjugation of the polysaccharide and carrier protein, the immunological compositions of the present invention are made by combining the various polysaccharide-protein conjugates, preferably in about equal amounts. The immunological compositions of the present invention comprise two or more different capsular polysaccharides conjugated to one or more carrier protein(s). A preferred embodiment of the present invention is a bivalent immunological composition comprising capsular polysaccharides from serogroups A and C *of N. meningitidis* each separately conjugated to diptheria toxoid.

The total amount of polysaccharide in the composition contains about 0.5 to about 50 µg polysaccharide, more preferably, about 2 to about 30 µg polysaccharide, and more preferably, about 5 to about 20 µg polysaccharide. The relative amounts of A and C polysaccharide in a given composition may vary, but preferably, are present in equal amounts within about 25% difference, more preferably, within about 15% difference, or alternatively in a range of A: C polysaccharide ratio of 1:3 to 3:1, more preferably, of a range of 1:2 to 2:1.

Preparation and use of carrier proteins, and a variety of potential conjugation procedures, are well known to those skilled in the art. Conjugates of the present invention can be prepared by such skilled persons using the teachings contained in the present invention as well as information readily available in the general literature. Guidance can also be obtained from any one or all of the following U.S. patents, the teachings of which are hereby incorporated in their entirety by reference: U.S. 4,356,170; U.S. 4,619,828; U.S. 5,153,312; U.S. 5,422,427 and U.S. 5,445,817.

The total amount of carrier protein in the composition contains about 20 to about 75 µg carrier protein, and more preferably, about 30 to about 50 µg carrier protein.

The immunological compositions of the present invention are made by separately preparing polysaccharide-protein conjugates from different meningococcal serogroups and then combining the conjugates. The immunological compositions of the present invention can be used as vaccines. Formulation of the vaccines of the present invention can be accomplished using art recognized methods. The vaccine compositions of the present invention may also contain one or more aluminum-free adjuvants. Adjuvants include, by way of example and not limitation, Freund's Adjuvant, BAY, DC-chol, pcpp, monophoshoryl lipid A, CpG, QS-21, cholera toxin and formyl methionyl peptide. See, e.g., Vaccine Design, the Subunit and Adjuvant Approach, 1995 (M.F. Powell and M. J. Newman, eds., Plenum Press, NY).

The present invention is directed to a method of inducing an immunological response in a patient, preferably a human patient.

As demonstrated below, the vaccines and immunological compositions according to the invention elicit a T-dependent-like immune response in various animal models, whereas the polysaccharide vaccine elicits a T-independent-like immune response. Thus, the compositions of the invention are also useful research tools for studying the biological pathways and processes involved in T-dependent-like immune responses to *N. meningitidis* antigens.

The amount of vaccine of the invention to be administered a human or animal and the regime of administration can be determined in accordance with standard techniques well known to those of ordinary skill in the pharmaceutical and veterinary arts taking into consideration such factors as the particular antigen, the adjuvant (if present), the age, sex, weight, species and condition of the particular animal or patient, and the route of administration. In the present invention, the amount of polysaccharide-protein carrier to provide an efficacious dose for vaccination against *N. meningitidis* can be from between about 0.02 µg to about 5 µg per kg body weight. In a preferred composition and method of the present invention the dosage is between about 0.1 µg to 3 µg per kg of body weight. For example, an efficacious dosage will require less antibody if the post-infection time elapsed is less since there is less time for the bacteria to proliferate. In like manner an efficacious dosage will depend on the bacterial load at the time of diagnosis. Multiple injections administered over a period of days could be considered for therapeutic usage.

The present invention provides a method for boosting in a human subject an anti-meningococcal immune response against a meningococcal capsular polysaccharides A and C. The method generally entails a primary vaccination using an aluminum-free polysaccharide-protein conjugate vaccine composition comprising meningococcal capsular polysaccharides A and C conjugated to a carrier protein e.g., A/C conjugate vaccine. In a preferred embodiment, a single primary vaccination is sufficient to elicit an anti-meningococcal immune response in the vaccinated subject which is specific for meningococcal serogroups A and C. After the immune response elicited by the primary vaccination has declined to sub-protective levels, a boosting vaccination is performed in order to provide a boosted anti-meningococcal immune response. The boosting vaccination may be a meningococcal A and C polysaccharide vaccine, or a meningococcal A and C conjugated to a carrier protein, e.g., A/C conjugate vaccine.

The multivalent conjugates of the present invention can be administered as a single dose or in a series (i.e., with a "booster" or "boosters"). For example, a child could receive a single dose early in life, then be administered a booster dose up to ten years later, as is currently recommended for other vaccines to prevent childhood diseases. Preferably, the patient is immunized in a single dose before one year of age. The present invention demonstrates that immunization with the A/C conjugate vaccine of the invention may be safely administered concomitantly with other childhood vaccines, such as DTP and OPV.

The booster dose will generate antibodies from primed B-cells, i.e., an anamnestic response. That is, the multivalent conjugate vaccine elicits a high primary (i.e., following a single administration of vaccine) functional antibody response in younger populations when compared to the licensed polysaccharide vaccine, and is capable of eliciting an anamnestic response (i.e., following a booster administration), demonstrating that the protective immune response elicited by the multivalent conjugate vaccine of the present invention is long-lived.

Compositions of the invention can include liquid preparations for orifice, *e.g.*, oral, nasal, anal, vaginal, peroral, intragastric, mucosal (*e.g.*, perlinqual, alveolar, gingival, olfactory or respiratory mucosa) etc., administration such as suspensions, syrups or elixirs; and, preparations for parenteral, subcutaneious, intradermal, intramuscular, intraperitoneal or intravenous administration (*e.g.*, injectable administration), such as sterile suspensions or emulsions. Intravenous and parenteral administration are preferred. Such compositions may be in admixture with a suitable carrier, diluent, or excipient such as sterile water, physiological saline, glucose or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, incorporated herein by reference, may be consulted to prepare suitable preparations, without undue experimentation.

Compositions of the invention are conveniently provided as liquid preparations, *e.g.*, isotonic aqueous solutions, suspensions, emulsions or viscous compositions that may be buffered to a selected pH. If digestive tract absorption is preferred, compositions of the invention can be in the "solid" form of pills, tablets, capsules, caplets and the like, including "solid" preparations which are time-released or which have a liquid filling, *e.g.*, gelatin covered liquid, whereby the gelatin is dissolved in the stomach for delivery to the gut. If nasal or respiratory (mucosal) administration is desired, compositions may be in a form and dispensed by a squeeze spray dispenser, pump dispenser or aerosol dispenser. Aerosols are usually under pressure by means of a hydrocarbon. Pump dispensers can preferably dispense a metered dose or a dose having a particular particle size.

Liquid preparations are normally easier to prepare than gels, other viscous compositions, and solid compositions. Additionally, liquid compositions are somewhat more convenient to administer, especially by injection or orally, to animals, children, particularly small children, and others who may have difficulty swallowing a pill, tablet, capsule or the like, or in multi-dose situations. Viscous compositions, on the other hand, can be formulated within the appropriate viscosity range to provide longer contact periods with mucosa, such as the lining of the stomach or nasal mucosa.

Obviously, the choice of suitable carriers and other additives will depend on the exact route of administration and the nature of the particular dosage form, *e.g.,* liquid dosage for (*e.g.*, whether the composition is to be formulated into a solution, a suspension, gel or another liquid form), or solid dosage form (*e.g.*, whether the composition is to be formulated into a pill, tablet, capsule, caplet, time release form or liquid-filled form).

Solutions, suspensions and gels, normally contain a major amount of water (preferably purified water) in addition to the active ingredient. Minor amounts of other ingredients such as pH adjusters (*e.g.*, a base such as NaOH), emulsifiers or dispersing agents, buffering agents, preservatives, wetting agents, jelling agents, (e.g., methylcellulose), colors and/or flavors may also be present. The compositions can be isotonic, *i.e*., it can have the same osmotic pressure as blood and lacrimal fluid.

The desired isotonicity of the compositions of this invention may be accomplished using sodium tartrate, propylene glycol or other inorganic or organic solutes. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount that will achieve the selected viscosity. Viscous compositions are normally prepared from solutions by the addition of such thickening agents.

A pharmaceutically acceptable preservative can be employed to increase the shelf life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the compositions must be selected to be chemically inert with respect to the *N. meningitidis* polysaccharide-protein carrier conjugates.

The invention will be further described by reference to the following illustrative, non-limiting examples setting forth in detail several preferred embodiments of the inventive concept. Other examples of this invention will be apparent to those skilled in the art without departing from the spirit of the invention.

### EXAMPLES

### Example 1 Preparation of Neisseria Meningitidis Serogroups A and C Purified Capsular Polysaccharide Powders

### Crude Paste Preparation

Separately, Neisseria meningitidis serogroup A and C wet frozen seed cultures are thawed and recovered with the aid of liquid Watson Scherp medium and planted in Blake bottles containing Mueller Hinton agar medium. The Blake are incubated at 35 to 37 deg. C. in a CO₂ atmosphere for 15 to 19 hours. Following the incubation period, the growth from the Blake bottles are dislodged and added to 4 L flasks containing Watson Scherp medium. The flasks are incubated at 35 to 37 deg. C. for 3 to 7 hours on a platform shaker. The contents of the 4 L flasks are transferred to a fermenter vessel containing Watson Scherp medium. The fermenter vessel is incubated at 35 to 37 deg. C. for 7 to 12 hours controlling dissolved oxygen content and pH with supplement feed and antifoam additions. After the incubation period, the contents of the fermentor vessel are transferred to a 500 L tank, Cetavlon^{™} is added, and the material mixed for 1 hours. The Cetavlon treated growth is centrifuged at approximately 15,000 to 17,000 x g at a flow rate of approximately 30 to 70 liters per hours. The crude polysaccharide is precipitated from the supernatant with a second Cetavlon^{™} precipitation. Cetavlon^{™} is added to the supernatant and the material mixed for at least 1 hour at room temperature. The material is stored at 1 to 5 deg. C. for 8 to 12 hours. The precipitated polysaccharide is collected centrifugation at approximately 45,000 to 50,000 x g at a flow rate of 300 to 400 ml per minute. The collected inactivated paste is stored at -60 deg. C. or lower until further processed. The inactivated paste may be prepared in several batches and combined.

### Purified Polysaccharide Powder Preparation

The inactivated paste is thawed and transferred to a blender. The paste is blended with 0.9 M calcium chloride to yield a homogeneous suspension. The suspension is centrifuged at approximately 10,000 x g for 15 minutes. The supernatant is decanted through a lint free pad into a container as the first extract. A second volume of 0.9 M calcium chloride is added to the paste, and blended to yield a homogeneous suspension. The suspension is centrifuged as above, and the supernatant combined with the supernatant from the first extraction. A total of four extractions are performed, and the supernatants pooled. The pooled extracts are concentrated by ultrifiltration using 10-30 kDA MWCO spiral would ultrafiltration units.

Magnesium chloride is added to the concentrated, and the pH adjusted to 7.2 to 7.5 using sodium hydroxide. DNase and RNase are added to the concentrate, and incubated at 25 to 28 deg. C. with mixing for 4 hours. Ethanol is added to a concentration of 30 to 50%. Precipitated nucleic acid and protein are removed by centrifugation at 10,000 x g for 2 hours. The supernatant is recovered and the polysaccharide precipitated by adding ethanol to 80% and allowing it to stand overnight at 1 to 5 deg. C. The alcohol is siphoned off, and the precipitated polysaccharide is centrifuged for 5 minutes at 10,000 x g. The precipitated polysaccharide is ished with alcohol. The polysaccharide is ished with acetone, centrifuged at 15 to 20 minutes at 10,000 x g. The polysaccharide is dried under vacuum. The initial polysaccharide powder is dissolved into sodium acetate solution. Magnesium chloride is added and the pH adjusted to 7.2 to 7.5 using sodium hydroxide solution. DNase and RNase are added to the solution and incubated at 25 to 28 deg. C. with mixing for 4 hours to remove residual nucleic acids. After incubation with these enzymes, an equal volume of sodium acetate-phenol solution is added to the polysaccharide-enzyme mixture, and placed on a platform shaker at 1 to 5 deg. C. for approximately 30 minutes. The mixture is centrifuged at 10,000 x g for 15 to 20 minutes. The upper aqueous layer is recovered and saved. An equal volume of sodium acetate-phenol solution is added to the aqueous layer, and extracted as above. A total of four extractions are performed to remove protein and endotoxin from the polysaccharide solution. The combined aqueous extracts are diluted up to ten fold with water for injection, and diafiltered against 10 volumes of water for injection. Calcium chloride is added to the diafiltered polysaccharide. The polysaccharide is precipitated overnight at 1 to 5 deg. C. by adding ethanol to 80%. The alcohol supernatant is withdrawn, and the polysaccharide collected by centrifugation at 10,000 x g for 15 minutes. The purified polysaccharide is ished two times with ethanol, and once with acetone. The ished powder is dried under vacuum in a desiccator. The dried powder is stored at -30 deg. C. or lower until processed onto conjugate.

### Example 2 Depolymerization of Neisseria Meningitidis serogroups A and C Purified Capsular Polysaccharide Powder

Materials used in the preparation include purified capsular polysaccharide powders from Neisseria meningitidis serogroups A and C prepared in accordance with the above Example, sterile 50 mM sodium acetate buffer, pH 6.0, sterile 1N hydrocholoric acid, sterile 1N sodium hydroxide, 30% hydrogen peroxide, and sterile physiological saline (0.85% sodium chloride). Alternatively, citrate buffer may be substituted for sodium acetate buffer.

Each serogroup polysaccharide is depolymerized in a separate reaction. A stainless steel tank is charged with up to 60 g of purified capsular polysaccharide powder. Sterile 50 mM sodium acetate buffer, pH 6.0 is added to the polysaccharide to yield a concentration of 2.5 g polysaccharide per liter. The polysaccharide solution is allowed to mix at 1 to 5 deg. C. for 12 to 24 hours to effect solution. The reaction tank is connected to a heat exchanger unit. Additional 50 mM sodium acetate buffer, pH 6.0, is added to dilute the polysaccharide to reaction concentration of 1.25 g per liter. The polysaccharide solution is heated to 55 deg. C..+-.0.1. An aliquot of 30% hydrogen peroxide is added to the reaction mixture to yield a reaction concentration of 1% hydrogen peroxide.

The course of the reaction is monitored by following the change in the molecular size of the polysaccharide over time. Every 15 to 20 minutes, aliquots are removed from the reaction mixture and injected onto a HPSEC column to measure the molecular size of the polysaccharide. When the molecular size of the polysaccharide reached the targeted molecular size, the heating unit is turned off and the polysaccharide solution rapidly cooled to 5 deg. C. by circulation through an ice water bath. The depolymerized polysaccharide solution is concentrated to 15 g per liters by connecting the reaction tank to an ultrafiltration unit equipped with 3000 MWCO regenerated cellulose cartridges. The concentrated depolymerized polysaccharide solution is diafiltered against about 5 to 15 volumes, preferably about 6 to 10 volumes, or more preferably, 10 volumes of sterile physiological saline (0.85% sodium chloride). The depolymerized polysaccharide is stored at 1 to 5 deg. C. until the next process step. The depolymerized polysaccharide may be prepared in batches and combined.

The preferred targeted size for the depolymerized polysaccharide is between about 5 and 75 kDa, preferably, between about 5 and 40 kDa, and more preferably, between about 10 and 25 kDa.

The molecular size of the depolymerized polysaccharide is determined by passage through a gel filtration chromatography column sold under the tradename "Ultahydrogel.TM.250" that is calibrated using dextran molecular size standards and by multi-angle laser light scattering. The quantity of polysaccharide is determined by phosphorus content for serogroup A using the method of Bartlet, G. R. J. (1959) Journal of Biological Chemistry, 234, pp-466-468, and by the sialic acid content for serogroups C, W135 and Y using the method of Svennerholm, L. (1955) Biochimica Biophysica Acta 24, pp604-611. The O-acetyl content is determined by the method of Hesterin, S. (1949) Journal of Biological Chemistry 180, p249. Reducing activity is determined by the method of Park, J. T. and Johnson, M. J. (1949 Journal of Biological Chemistry 181, pp149-151. The structural integrity of the depolymerized polysaccharide is determined by protein .sup.1H and .sup.13C NMR. The purity of the depolymerized polysaccharide is determined by measuring the LAL (endotoxin) content and the residual hydrogen peroxide content.

### Example 3 Derivatization of Neisseria Meningitidis Serogroups A, C, W-135, and Y Depolymerized Polysaccharide

Materials used in this preparation include hydrogen peroxide, depolymerized capsular polysaccharide serogroups A and C from Neisseria meningitidis, prepared in accordance with the above Example 2, adipic acid dihydrazide, 1-ethyl-3-(3-dimethylaminopropyl) carbodiimide (EDAC) for serogroup A only, sodium cyanborohydride, sterile 1N hydrocholoric acid, sterile 1N sodium hydroxide, sterile 1 M sodium chloride, and sterile physiological saline (0.85% sodium chloride).

Each serogroup polysaccharide is derivatized in a separate reaction. A stainless steel tank is charged with the purified depolymerized polysaccharide, and diluted with sterile 0.85% physiological saline to achieve a final reaction concentration of 6 g polysaccharide per liter. To this solution is added a concentrated aliquot of adipic acid dihydrazide dissolved in sterile 0.85% physiological saline, in order to achieve a reaction concentration of lg per liter. For serogroup A only, EDAC is added as a concentrated aliquot dissolved in sterile 0.85% physiological saline, to achieve a reaction concentration of lg per liter. The pH is adjusted to 5.0.+-.0.1, and this pH is maintained for 2 hours using sterile 1N hydrochloric acid and sterile 1N sodium hydroxide at room temperature (15 to 30 deg. C.). After two hours, a concentrated aliquot of sodium cyanoborohydride, dissolved in 0.85% physiological saline, is added to the reaction mixture to achieve a reaction concentration of 2 g per liter. The reaction is stirred at room temperature (15 to 30 deg. C.) for 44 hours.+-.4 hours while maintaining the pH at 5.5.+-.0.5. Following this reaction period, the pH is adjusted to 6.0.+-.0.1, and the derivatized polysaccharide is concentrated to 12 g polysaccharide per liter by connecting the reaction tank to a ultrafiltration unit equipped with a 3000 MWCO regenerated cellulose cartridges. The concentrated derivatized polysaccharide is diafiltered against 30 volumes of 1 M sodium chloride, followed by 10 volumes of 0.15 M sodium chloride. The tank is disconnected from the ultrafiltration unit and stored at 1 to 5 deg. C. for 7 days. The tank is reconnected to an ultrafiltration unit equipped with 3000 MWCO regenerated cellulose cartridges, and diafiltered against 30 volumes of 1 M sodium chloride, followed by 10 volumes of 0.15 M sodium chloride. Alternatively, the concentrated derivatized polysaccharide is dialyzed against about 10 to about 30 volumes 1M sodium chloride and then against 10 to about 30 volumes physiological saline.

The molecular size of the derivatized polysaccharide, the quantity of polysaccharide, and the O-acetyl content are measured by the same methods used on the depolymerized polysaccharide. The hydrazide content is measured by the 2,4,6-trinitrobenzensulfonic acid method of Snyder, S. L. and Sobocinski, P. Z. (1975) Analytical Biochemistry 64, pp282-288. The structural integrity of the derivatized polysaccharide is determined by proton ¹H and ¹³C NMR. The purity of the derivatized polysaccharide is determined by measuring the level of unbound hydrazide, the LAL (endotoxin) content, and the residual cyanoborohydride content.

### Example 4 Preparation of Carrier Protein

### Preparation of Crude Diphtheria Toxoid Protein

Lyophilized seed cultures are reconstituted and incubated for 16 to 18 hours. An aliquot from the culture is transferred to a 0.5-liter flask containing growth medium, and the culture flask is incubated at 34.5 to 36.5 deg. C. on a rotary shaker for 7 to 9 hours. An aliquot from the culture flask is transferred to a 4-liter flask containing growth medium, and the culture flask is incubated at 34.5 to 36.5 deg. C. on a rotary shaker for 14 to 22 hours. The cultures from the 4-liter flask are used to inoculate a fermenter containing growth media. The fennenter is incubated at 34.5 to 36.5 deg. C. for 70 to 144 hours. The contents of the fermenter are filtered through depth filters into a collection vessel. An aliquot of formaldehyde solution, 37% is added to the harvest to achieve a concentration of 0.2%. The pH is adjusted to 7.4 to 7.6. The harvest is filtered through a 0.2 micron filter cartridge into sterile 20 liter bottles. The bottles are incubated at 34.5 to 36.5 deg. C. for 7 days. An aliquot of formaldehyde solution, 37%, is added to each 20 liter bottle to achieve a concentration of 0.4%. The pH of the mixtures is adjusted to 7.4 to 7.6. The bottles are incubated at 34.5 to 36.5 deg. C. for 7 days on a shaker. An aliquot of formaldehyde solution, 37%, is added to each 20 liter bottle to achieve a concentration of 0.5%. The pH of the mixtures is adjusted to 7.4 to 7.6. The bottles are incubated at 34.5 to 36.5 deg. C. for 8 weeks. The crude toxoid is tested for detoxification. The bottles are stored at 1 to 5 deg. C. during the testing period.

### Purification of the Crude Diphtheria Toxoid Protein

The crude toxoid is allowed to warm to room temperature, and the contents of the 20-liter bottles are combined into a purification tank. The pH of the toxoid is adjusted to 7.2 to 7.4, and charcoal is added to the crude toxoid and mixed for 2 minutes. The charcoal toxoid mixture is allowed to stand for 1 hours, and is then filtered through a depth filter cartridge into a second purification tank. Solid ammonium sulfate is added to the filtrate to achieve 70% of saturation. The pH is adjusted to 6.8 to 7.2, and the solution is allowed to stand for 16 hours. The precipitated protein is collected by filtration and ished with 70% of saturation ammonium sulfate solution, pH 7.0. The precipitate is dissolved into sterile distilled water, and the protein solution is filtered into a stainless steel collection vessel. The pH is adjusted to 6.8 to 7.2, and ammonium sulfate is added to 40% of saturation. The pH of the solution is adjusted to 7.0 to 7.2, and the solution is allowed to stand for 16 hours. The precipitate is removed by filtration and discarded. Ammonium sulfate is added to the filtrate to 60% of saturation, and the pH adjusted to 7.0 to 7.2. The mixture is allowed to stand for 16 hours, and the precipitated protein is collected by filtration. The precipitate is dissolved into sterile distilled water, filtered to remove undissolved protein, and diafiltered against 0.85% physiological saline.

### Concentration and Sterile Filtration of the Purified Diphtheria Toxoid Protein

The protein solution is concentrated to 15 g per liter and diafiltered against 10 volumes of 0.85% physiological saline suing a 10,000 MWCO regenerated cellulose filter cartridge. The concentrated protein solution is sterilized by filtration through a 0.2 micron membrane. The protein solution is stored at 1 to 5 deg. C. until processed onto conjugate.

The protein concentration is determined by the method of Lowry, O. H. et. al (1951) Journal of Biological Chemistry 193, p265-275. The purity of the protein is measured by sterility, LAL (endotoxin) content, and residual formaldehyde content.

### Example 5 Preparation of Monovalent Conjugates of Neisseria Meningitidis Serogroups A and C Polysaccharide to Diphtheria Toxoid Protein

Materials used in this preparation include adipic acid derivatized polysaccharide from Neisseria meningitidis serogroups A and C, prepared in accordance with the above Example, sterile diphtheria toxoid protein, prepared in accordance with the above Example, EDAC, ammonium sulfate, sterile 1N hydrochloric acid, sterile 1N sodium hydroxide, and sterile physiological saline (0.85%).

Each serogroup polysaccharide conjugate is prepared by a separate reaction. All four conjugates are prepared by the following process. A stainless steel tank is charged with the purified adipic acid derivatized polysaccharide at a reaction concentration of 700 to 1000 .mu.moles of reactive hydrazide per liter and purified diphtheria toxoid protein at a reaction concentration of 3.8 to 4.0 g protein per liter. Physiological saline 0.85%, is used to dilute the starting materials to the target reaction concentrations and the pH is adjusted to 5.0.+-.0.1. An aliquot of EDAC is added to the polysaccharide protein mixture to achieve a reaction concentration of 2.28 to 2.4 g per liter. The pH of the reaction is kept at 5.0.+-.0.1 for 2 hours at 15 to 30 deg. C. After two hours, the pH is adjusted to 7.0.+-.0.1 using sterile 1N sodium hydroxide, and the reaction is stored at 1 to 5 deg. C. for 16 to 20 hours.

The reaction mixture is allowed to warm to 15 to 30 deg. C. and the reaction vessel is connected to an ultrafiltration unit equipped with a 30,000 MWCO regenerated cellulose cartridge. For serogroup A, solid ammonium sulfate is added to 60% of saturation, and for serogroup C, solid ammonium sulfate is added to 50% of saturation. For serogroups A, the conjugate reaction mixture is diafiltered against 20 volumes of 60% of saturated ammonium sulfate solution, and for serogroup C, the conjugate reaction mixture is diafiltered against 20 volumes of 50% of saturated ammonium sulfate solution, followed by 20 volumes of physiological saline, 0.85%. The diafiltered conjugate is first filtered through a filter capsule containing a 1.2 micron and a 0.45 micron filter, and then through a second filter capsule containing a 0.22 micron filter. Alternatively, the conjugate reaction mixture may be purified by several, preferably about three, ammonium sulfate precipitations.

The quantity of polysaccharide and O-acetyl content are measured by the same methods used on the depolymerized and derivatized polysaccharide. The quantity of protein is determined by the Lowry method. The molecular size of the conjugate is determined by passage through a gel filtration chromatography column sold under the tradename "TSK6000PW" that used DNA as the void volume marker, ATP as the total volume marker, and bovine thyroglobulin as a reference marker. In addition, the molecular size of the conjugate eluted from the TKS6000PW column is measured by multi-angle laser light scattering. The antigenic character of the conjugate is measured by binding to anti-polysaccharide serogroup specific antibody using double-sandwich ELISA method. The purity of the conjugates is determined by measuring the amount of unbound (unconjugated) polysaccharide by elution though a hydrophobic interaction chromatography column, unconjugated protein by capillary electrophoresis, sterility, LAL (endotoxin) content, residual EDAC content, and residual ammonium ion content.

### Example 6 Formulation of an aluminum-free multivalent meningococcal A and C polysaccharide diphtheria toxoid conjugate vaccine

Materials used in preparing a meningococcal A and C conjugates may be prepared in accordance with the above methods. Preferably, the vaccine composition is formulated in sterile pyrogen-free, phosphate buffered physiological saline. The saline concentration may be achieved by 0.9% of 15 mM sodium chloride and 10 mM sodium phosphate. Preferably, the vaccine composition does not contain aluminum.

### Example 7 Immunogenicity of an aluminum-free multivalent meningococcal A and C polysaccharide diphtheria toxoid conjugate vaccine in Human Patients

A clinical study is performed with infant subjects that compared the immune response to the bivalent A/C polysaccharide vaccine versus the bivalent A/C conjugate vaccine. In this study, a third group of infants are enrolled to serve as a control group and they received a *Haemophilus influenzae* type b conjugate. All three vaccine groups receive the same pediatric vaccines. The bivalent A/C conjugate group received three doses of diptheria conjugate vaccine (4 µg polysaccharide per dose) at 6, 10, and 14 weeks of age. The bivalent A/C polysaccharide group received two doses of a bivalent AC polysaccharide vaccine (50 µg polysaccharide per dose) at 10 and 14 weeks of age. The *Haemophilus influenzae* type b conjugate group received three doses of conjugate vaccine at 6,10, and 14 weeks of age. Blood specimens are taken at 6 weeks, pre-vaccination, and at 18 weeks, 4 weeks post vaccination. When the children are 11 to 12 months of age, blood specimens are taken and the children who had received either the bivalent AC conjugate or the bivalent AC polysaccharide vaccine received a booster dose of AC polysaccharide. The reason for the booster dose of polysaccharide is to evaluate whether or not the subjects would elicit an anemestic response.

The results of this study, both the primary and polysaccharide booster immune responses are presented in Table 1 for the IgG antibody response and Table 2 for the SBA antibody response. The IgG antibody response post primary series is approximately the same for both the polysaccharide and conjugate vaccine. However, the bactericidal antibody response in the conjugate vaccinated subjects is much higher than that for the polysaccharide vaccinated subjects. As observed with the one year old subjects, vaccination of infants with the polysaccharide elicits very little functional-bactericidal antibody. The antibody elicited by the infants to the polysaccharide vaccine is presumably low avidity antibody, whereas, the conjugate vaccine appears to elicit high avidity antibody, thereby accounting for the much higher titer of bactericidal antibody. The high level of functional antibody elicited by the booster dose of polysaccharide vaccine in the subjects who had received the conjugate vaccine in the primary vaccination series, indicates that these subjects have been primed for a memory or T-cell dependent antibody response. The subjects who received the polysaccharide vaccine in the primary vaccination series elicited a modest response to the polysaccharide booster dose, that is indicative of a T-cell independent response.

Table 1 shows anti-polysaccharide IgG GMC (group mean concentration) in infants against serogroups A and C before and after both the primary series immunization (6, 10 and 14 weeks of age) and the booster vaccination with bivalent AC polysaccharide given at 11 to 12 months of age.

**Table 1**

| **Immune Response by Vaccine Group** | **Primary Vaccination GMC [95% CI]** | | | **PS Booster Vaccination GMC [95% CI]** | | | |
|---|---|---|---|---|---|---|---|
| | **N** | **Pre** | **Post** | **N** | **Pre** | | **Post** |
| **Serogroup A:** | | | | | | | |
| AC Conjugate | 34 | 3.4 [2.2-5.4] | 5.8 [4.3-8.0] | 31 | 0.2 [0.1-0.3] | | 7.0 [4.0-12.0] |
| AC Polysaccharide | 35 | 3.0 [1.7-5.3] | 5.5 [4.1-7.3] | 30 | 0.9 [0.5-1.4] | | 3.1 [2.0-4.7] |
| HIB Conjugate | 36 | 3.2 [2.2-4.5] | 0.6 [0 4-0.8] | NA | NA | | NA |

| **Serogroup C:** | | | | | | | |
|---|---|---|---|---|---|---|---|
| AC Conjugate | 31 | 1.6 [0.9-2.8] | 2.8 [2.0-3.9] | 31 | 0.1 [0.1-0.2] | | 8.1 [4.5-14.5] |
| AC Polysaccharide | 35 | 2.3 [1.4-3.9] | 5.3 [3.8-7.4] | 30 | 0.6 [0.3-1.0] | | 2.8 [1.7-4.7] |
| HIB Conjugate | 36 | 2.0 [1.2-3.5] | 0.5 [0.3-0.7] | NA | NA | | NA |

Table 2 shows SBA antibody GMT (group mean titer) in infants against serogroups A and C before and after both the primary series immunization (6, 10 and 14 weeks of age) and booster vaccination with bivalent AC polysaccharide given at 11 to 12 months of age.

**Table 2**

| **Immune Response By Vaccine Group** | **Primary Vaccination GMT [95% CI]** | | | | | **PS Booster Vaccination GMT[95% CI]** | | | | |
|---|---|---|---|---|---|---|---|---|---|---|
| | **N** | **Pre** | | **Post** | | **N** | **Pre** | | **Post** | |
| **Serogroup A:** | | | | | | | | | | |
| AC Conjugate | 34 | 11.8 [7.2-19.3] | | 177 [101-312] | | 24 | 10.1 [5.6-18.0] | | 373 [162-853] | |
| AC Polysaccharide | 32 | 14.7 [8.5-25.4] | | 7.0 [4.7-10.5] | | 26 | 6.1 [3.9-9.5] | | 24.1 [11-53] | |
| HIB Conjugate | 35 | 11.2 [6.8-18.3] | | 6.7 [4.3-10.5] | | NA | NA | | NA | |

| **Serogroup C:** | | | | | | | | | | |
|---|---|---|---|---|---|---|---|---|---|---|
| AC Conjugate | 34 | 50.8 [24-107] | | 189 [128-278] | | 27 | 4.6 [3.6-5.6] | | 287 [96.2-858] | |
| AC Polysaccharide | 32 | 62.7 [29-131] | | 25.4 [14.4-44.6] | | 26 | 4.1 [3.9-4.3] | | 14.4 [7.9-26.1] | |
| HIB Conjugate | 36 | 45.3 [21.9-133] | | 7.3 [4.7-11.3] | | NA | NA | | NA | |

In addition to the benefits that this invention offers to the improved protection against meningococcal disease in young populations and the wider protection against serogroups A, C, W-135 and Y, the tetravalent conjugate may provide protection to other pathogens by inducing an antibody response to the carrier protein. When the tetravalent conjugate vaccine, using diphtheria toxoid conjugate, is administered to infants, these subjects also received the routine pediatric immunizations, which included diphtheria toxoid. Therefore, in these subjects there is no apparent improvement in the antibody response to diphtheria toxoid. However, when the diphtheria toxoid conjugate is administered to subjects that did not receive concomitant diphtheria toxoid containing vaccines, a strong booster response to diphtheria toxoid is observed. These subjects had received a three dose regiment of DTP at 2, 3, and 4 months of age. In this study, the subjects received either single dose of a bivalent AC conjugate or a single dose of bivalent AC polysaccharide vaccine between 2 and 3 year of age. Blood specimens are taken at the time of vaccination and 30-days post vaccination. The bivalent AC conjugate used diphtheria toxoid as the carrier protein.

The immune response of diphtheria toxoid in the two vaccine groups is presented in Table 3. The polysaccharide did not serve to stimulate an anti-diphtheria immune response in these subjects as expected, however a strong anti-diphtheria immune response is observed for the subjects receiving the AC conjugate. Therefore, the meningococcal conjugate vaccine may provide an added benefit of stimulating an immune response to carrier protein thereby providing protection against diseases caused by *Corynebacteria diphtheriae* when diphtheria toxoid is used as a carrier protein.

Table 3 shows anti-diphtheria antibody by ELISA GMT (group mean titer) in IU/ml in young healthy children vaccinated with either a bivalent AC diphtheria toxoid conjugate vaccine formulated at 4 µg as polysaccharide per dose or a bivalent AC polysaccharide vaccine formulated at 50 µg as polysaccharide per dose

**Table 3**

| **Immune Response by Vaccine Group** | **Nₚᵣₑ/Nₚₒₛₜ** | **Anti-Diphtheria Antibody (ELISA -IU/ml) [95%CI]** | |
|---|---|---|---|
| | | **Pre** | **Post** |
| AC Conjugate | 104/103 | 0.047 [0.036 - 0.060] | 21.2 [11.6 - 38.6] |
| AC Polysaccharide | 103/102 | 0.059 [0.045 - 0.076] | 0.059 [0.045 - 0.077] |

### Example 8 Immunogenicity, safety, and memory of different schedules of an unadjuvanted Neisseria meningitidis A/C-diphtheria toxoid conjugate vaccine in infants

A clinical study in an open-label, randomized controlled trial of 618 infants in Niger receiving one to four doses of a vaccine of polysaccharides A and C conjugated to diphtheria toxoid, (A/C Conjugate) or a standard A/C polysaccharide (A/C PS) vaccine simultaneous with routine infant immunizations is presented. At 24 months, A/C PS vaccine is given and memory response measured one week later. Serum bactericidal activity (SBA) and IgG antibody by ELISA are measured.

The vaccine comprised capsular polysaccharides of *N. meningitidis* serogroups A and C conjugated to diphtheria toxoid. The vaccine is in a 0.5 ml disposable syringe containing 4 µg of each of the two polysaccharides conjugated to 48 µg of diphtheria toxoid. The unadjuvanted A/C conjugate vaccine is formulated into a dose of 0.5 mL of pyrogen-free, phosphate buffered physiological saline with no preservative, specifically, 0.9% of 15 mM sodium chloride and 10 mM sodium phosphate.

The 618 infants enrolled in the study are randomized into 6 groups of equal size. Inclusion criteria are: 1) infant in good health with rectal temperature <38°C; 2) between 5 and 11 weeks of age; 3) delivered at >36 weeks gestation; 4) family resided permanently in Niamey and 5) parents providing written consent. Exclusion criteria are: severe chronic illness; enrolled in another clinical trial; previously vaccinated with DTP vaccine, Meningococcal PS, or *Haemophilus influenzae b* (Hib) conjugate vaccine; preceding meningitis; administration of BCG or corticosteroid therapy within the past 3 weeks; or a contraindication to vaccination.

Children randomized to the control groups received either Meningococcal A/C polysaccharide (MenPS, Aventis Pasteur) that contained 50 µg of each polysaccharide, or Hib conjugate vaccine (Act-Hib, Aventis Pasteur). Intramuscular injections of MenD, MenPS and Act-Hib are given in the anterolateral right thigh. Children had received BCG and oral polio vaccine (OPV) at birth. In accordance with the Expanded Program on Immunization (EPI) schedule, they received DTP and OPV at 6, 10, and 14 weeks, with boosters at 15 months. Measles and yellow fever vaccines are given at age 9 months. EPI injections are given intramuscularly in the left deltoid muscle.

There are 6 groups of 103 infants who received four (Group 1), three (group 2), two (group 3), or one dose (groups 4 and 5) of A/C Conjugate or one dose of A/C PS (group 6) during the first 9 months of life concomitant with routine EPI vaccines, see Table 4 below:

**Table 4**

| **Group assignments, trial schedule, and number of subjects with evaluable blood specimens** | | | | | | | | | |
|---|---|---|---|---|---|---|---|---|---|
| | **Visit 1** 6 weeks | **Visit 2** 10 weeks | **Visit 3** 14 weeks | **Visit 4** 18 weeks | **Visit 5** 9 months | **Visit 6** 10 months | **Visit 7** 15 months | **Visit 8** 24 months | **Visit 9** 24 months + 1 week |
| Group 1 N=104 | MenD | MenD | MenD | N=98 | MenD | N=99 | | MenPS N=84 | N=77 |
| Group 2 N=103 | MenD | MenD | MenD | N=97 | | N=92 | | MenPS N=85 | N=74 |
| Group 3 N=103 | | | MenD | N=94 | MenD | N=89 | | MenPS N=73 | N=66 |
| Group 4 N=103 | | | MenD | N=97 | | N=93 | | MenPS N=83 | N=70 |
| Group 5 N=103 | Act-Hib | Act-Hib | Act-Rib | N=101 | MenD | N=97 | | MenPS N=87 | N=76 |
| Group 6 N=102 | Act-Hib | Act-Hib | Act-Hib | N=97 | MenPS | N=88 | | MenPS N=82 | N=73 |
| Other vaccines | DTP OPV | DTPOPV | DTP OPV | | Yellow fever Measles | | DTP OPV | | |
| Specimens *all groups* | | | | Blood sample | | Blood sample | | Blood sample | Blood sample |

At 24 months of age, subjects received a dose of A/C PS, in order to evaluate the anamnestic response and simulate immune response on encountering *N. meningitidis* bacteria. Four 3 mL of blood specimens are collected, at 18 weeks of age, 10 months, 24 months, and one week later.

Children are monitored for 30 minutes after each injection for immediate reactions that might represent hypersensitivity reactions. Follow up evaluations are made during home visits 24 and 72 hours after study injections.

Serum bactericidal activity is measured for both A and C serogroups by the standard methods using baby rabbit complement, Maslanka SE, et al., Clin Diagn Lab Immunol 1997; 4: 155-67. Bactericidal activity is defined as the reciprocal serum dilution yielding ≥ 50% of bacterial growth in comparison to a control culture. IgG concentration is measured by the standardized ELISA and expressed in µg/mL, Carlone GM, et al., J Clin Microbiol 1992; 30: 154-9' and Gheesling LL, et al., J Clin Microbiol, 1994; 32: 1475-82. Antibodies against diphtheria, tetanus, pertussis, and polio virus types 1, 2, and 3 in serum from 18 weeks of age, are measured using standard methods. Reactogenicity is evaluated for each study group following each dose administered based on the proportion of infants who had at least one local reaction within 30 minutes of administration, or one local or systemic reaction within 24 or 72 hours following administration.

Immune response is expressed as antibody concentrations for ELISA and geometric mean titers (GMT) of the inhibitory dilution for SBA. Antibody levels have been considered protective based on ≥ 2 µg/mL according to ELISA and ≥ 1:4 for SBA using human complement, Goldschneider I, et al., J. Exp. Med., 1969, 129: 1327-48 and Lepow ML, et al., Pediatrics 1977; 60: 673-680. In addition, the results present the percent of infants with ELISA antibody concentration ≥2 µg/mL and an SBA titer of ≥ 1:128, Jodar L, et al., Biologicals 2002; 30: 323-9. Confidence intervals are calculated for GMT and antibody concentrations.

Groups are compared by ANOVA analysis of variance according to the distribution of log titers for SBA against serogroup A and C at visit 6. The Student-Newman-Keuls test is used for multiple comparisons. The anamnestic response is evaluated by comparison of percentages and 95% confidence intervals and the ratio of GMTs of infants with serologic protection compared with the baseline of SBA and ELISA for group 6. No severe adverse event is attributed to vaccine given by the study.

Table 5 shows the SBA titers for serogroups A and C at 18 weeks, 10 months, 24 months and one week after 24 month time period are provided in Table 8, below, for the six groups. The proportion of subjects having SBA titers ≥ 1:128 are provided for each of the Groups.

**Table 5**

| **Serum bactericidal activity serogroup A and C polysaccharides** | | | | | | | | |
|---|---|---|---|---|---|---|---|---|
| | Visit 4 (18 weeks) | | Visit 6 (10 months) | | ' Visit 8 (24 months) | | Visit 9 (24 months + 1 week) | |
| | GMT (n) | % ≥1/128 | GMT (n) | % ≥ 1/128 | GMT (n) | % ≥ 1/128 | GMT (n) | % ≥ 1/128 |
| **Serogroup A** | | | | | | | | |
| Group 1 | 87.4 (55) [62.2 - 117] | 56.1 [45.7 - 66.1] | 309 (78) [229 - 417] | 88.6 [80.1 - 94.4] | 48.3 (32) [30.6 - 76.4] | 38.1 [27.7 - 49.3] | 3351 (76) [2585 - 4345] | 100 [95.3 - 100] |
| Group 2 | 84.6 (54) [61.4 - 117] | 55.7 [45.2 - 65.8] | 7.65 (6) [5.88 - 9.94] | 6.5 [2.4 - 13.7] | 35.3 (33) [21.8 - 57.0] | 38.8 [28.4 - 50.0] | 1421 (71) [978 - 2066] | 95.9 [88.6 - 99.2] |
| Group 3 | 152 (64) [107 - 215] | 68.1 [57.7 - 77.3] | 415 (76) [302 - 570] | 85.4 [76.3 - 92.0] | 81.1 (35) [49.5 - 133] | 47.9 [36.1 - 60.0] | 2761 [2182 - 3492] | 100 (65) [94.5 - 100] |
| Group 4 | 98.3 (59) [69.3 - 139] | 60.8 [50.4 - 70.6] | 8.37 (4) [6.66 - 10.51 | 4.3 [1.2 - 10.8] | 55.5 (37) [33.2 - 92.9] | 44.6 [33.7 - 55.9] | 2376 (70) [1809 - 3121] | 100 [94.9 - 100] |
| Group 5 | 5.19(3) [4.38 - 6.16] | 3.0 [0.6 - 8.4] | 129 (60) [84.2 - 197] | 61.9 [51.4 - 71.5] | 69.3 (42) [41.8 - 115] | 48.3 [37.4- 59.2] | 2549 (76) [1913 - 3397] | 100 [95.3 - 100] |
| Group 6 | 4.65 (2) [4.08 - 5.29] | 2.1 [0.3 - 7.3] | 7.22 (4) [5.63 - 9.27] | 4.5 [1.3 - 11.2] | 32.8 (30) [20.1 - 53.7] | 36.6 [26.2 - 48.0] | 1250 (71) [883 - 1770] | 97.3 [90.5 - 99.7] |

| **Serogroup C** | | | | | | | | |
|---|---|---|---|---|---|---|---|---|
| Group 1 | 289 (82) [205 - 406] | 83.7 [74.8 - 90.4] | 215 (64) [138 - 337] | 72.7 [62.2 - 81.7] | 8.41 (8) [6.01 - 11.8] | 9.5 [4.2 - 17.9] | 711 (72) [482 - 1049] | 94.7 [87.1 - 98.5] |
| Group 2 | 304 (83) [222 - 415] | 85.6 [77.0 - 91.9] | 6.98 (8) [5.43 - 8.97] | 8.8 [3.9 - 16.6] | 12.7 (19) [8.22 - 19.7] | 22.4 [14.0 - 32.7] | 617 (61) [383 - 996] | 82.4 [71.8 - 90.3] |
| Group 3 | 111 (60) [74.4 - 166] | 63.8 [53.3 - 73.5] | 553 (76) [373 - 821] | 85.4 [76.3 - 92.0] | 16.6 (18) [9.68 - 28.5] | 24.7 [15.3 - 36.1] | 1655 (62) [1064 - 2574] | 95.4 [87.1 - 99.0] |
| Group 4 | 79.9 (55) [53.7 - 119] | 56.7 [46.3 - (7) 66.7] | 6.58 [5.32 - 8.13] | 7.6 [3.1 - 15.1] | 16.8 (20) [10.3 - 27.4] | 24.1 [15.4 - 34.7] | 1855 (65) [1146 - 3003] | 92.9 [84.1 - 97.6] |
| Group 5 | 6.83 (7) [5.41 - 8.62] | 6.9 [2.8 - 13.8] | 19.1 (25) [13.4 - 27.3] | 25.8 [17.4 - 35.7] | 13.4 (19) [8.53- 21.1] | 21.8 [13.7 - 32.0] | 2244 (75) [1579 - 3188] | 98.7 [92.9 - 100] |
| Group 6 | 5.29 (2) 14.44- 6.30] | 2.1 [0.3 - 7.3] | 9.97 (10) [7.31- 13.6] | 11.4 [5.6 - 19.9] | 5.33 (3) [4.41 - 6.45] | 3.7 [0.8 - 10.3] | 68.4(38) [42.1 - 111] | 52.1 [40.0 - 63.9] |

| | | | | | | | | |
|---|---|---|---|---|---|---|---|---|
| GMT geometric mean titer, [95% confidence interval] | | | | | | | | |

Table 6 shows ELISA results for each Group.

**Table 6**

| **ELISA antibody concentrations to group A and C polysaccharide** | | | | | | | | |
|---|---|---|---|---|---|---|---|---|
| | **Visit 4 (18 weeks)** | | **Visit 6 (10 months)** | | **Visit 8 (24 months)** | | **Visit 9 (24 mos + 1 wk)** | |
| | GMC | % ≥ 2µg·mL⁻¹ | GMC | % ≥ 2µg·ml⁻¹ | GMC | % ≥ 2µg·mL⁻¹ | GMC | % ≥ 2µg·mL⁻¹ |
| **Serogro up A** | | | | | | | | |
| **Group 1** | 3.84 [3.37 - 4.38] | 84.7 [76.0 - 91.2] | 3.01 [2.42 - 3.74] | 67.0 [56.2 - 76.7] | 0.35 [0.27 - 0.46] | 8.3 [3.4 - 16.4] | 10.0 [7.80 - 12.9] | 98.7 [92.9 - 100] |
| **Group 2** | 3.90 [3.34- 4.56] | 75.3 [65.5 - 83.5] | 0.24 [0.20 - 0.291 | 0 [0-3.9] | 0.39 [0.27- 0.56] | 21.2 [13.1 - 31.4] | 6.78 [5.21- 8.83] | 87.8 [78.2 - 94.3] |
| **Group 3** | 4.82 [4.05 - 5.73] | 86.2 [77.5 - 92.4] | 3.68 [2.92 - 4.64] | 71.9 [61.4 - 80.9] | 0.66 [0.43 - 1.01] | 27.4 [17.6 - 39.1] | 12.0 [9.30 - 15.6] | 92.3 [83.0 - 97.5] |
| **Group 4** | 3.87 [3.27 - 4.58] | 80.4 [71.1 - 87.8] | 0.25 [0.21 - 0.29] | 1.1 [0 - 5.9] | 0.46 [0.31 - 0.67] | 20.5 [12.4 - 30.8] | 9.04 [6.98 - 11.7] | 91.4 [82.3 - 96.8] |
| **Group 5** | 0.42 [0.33 - 0.53] | 9.9 [4.9 - 17.5] | 2.47 [1.93 - 3.18] | 60.8 [50.4 - 70.6] | 0.52 [0.35 - 0.77] | 20.7 [12.7 - 30.7] | 13.2 [10.3 - 17.0] | 93.4 [85.3 - 97.8] |
| **Group 6** | 0.47 [0.38 - 0.59] | 10.3 [5.1 - 18.1] | 1.64 [1.28 - 2.10] | 44.3 [33.7 - 55.3] | 0.56 [0.41 - 0.77] | 20.7 [12.6 - 31.1] | 5.74 [4.49 - 7.34] | 82.2 [71.5 - 90.2] |

| **Serogro up C** | | | | | | | | |
|---|---|---|---|---|---|---|---|---|
| **Group 1** | 9.34 [7.98 - 10.9] | 96.9 [91.3 - 99.4] | 6.70 [5.27 - 8.50] | 85.2 [76.1 - 91.9] | 0.44 [0.32 - 0.60] | 11.9 [5.9 - 20.8] | 9.79 [6.74 - 14.20] | 76.3 [65.2 - 85.3] |
| **Group 2** | 9.45 [7.88 - 11.3] | 92.8 [85.7 - 97.0] | 0.79 [0.63 - 0.99] | 23.9 [15.6 - 33.9] | 0.64 [0.42 - 0.96] | 27.1 [18.0 - 37.8] | 9.73 [6.82 - 13.9] | 82.4 [71.8 - 90.3] |
| **Group 3** | 5.59 [4.60 - 6.78] | 84.0 [75.0 - 90.8] | 8.62 [6.70 - 11.1] | 87.6 [79.0 - 93.7] | 0.73 [0.48 - 1.11] | 24.7 [15.3 - 36.1] | 15.8 [11.6 - 21.6] | 92.3 [83.0 - 97.5] |
| **Group 4** | 4.38 [3.60 - 5.33] | 79.4 [70.0 - 86.9] | 0.68 [0.55 - 0.84] | 15.2 [8.6 - 24.2] | 0.48 [0.34 - 0.67] | 15.7 [8.6 - 25.3] | 17.0 [12.5 - 23.2] | 94.3 [86.0 - 98.4) |
| **Group 5** | 0.38 [0.30 - 0.49] | 9.9 [4.9 - 17.5] | 1.95 [1.58 - 2.41] | 50.5 [40.2 - 60.8] | 0.31 [0.21 - 0.44] | 18.4 [10.9 - 28.1] | 9.18 [7.03 - 12.0] | 90.8 [81.9 - 96.2] |
| **Group 6** | 0.33 [0.27 - 0.41] | 4,1 [1.1 - 10.2] | 7.84 [6.45 - 9.52] | 90.9 [82.9 - 96.0] | 0.48 [0.35 - 0.64] | 13.4 [6.9 - 22.7] | 2.61 [1.93;3.5 3] | 54.8 [42.7 - 66.5] |

| | | | | | | | | |
|---|---|---|---|---|---|---|---|---|
| GMC geometric mean concentrations, [95% confidence interval] | | | | | | | | |

### Response to EPI vaccinations

There is no difference in antibody concentrations against the EPI vaccines (diphtheria, tetanus, polio virus 1,2, and 3, pertussis) between the 6 groups. The results are provided below in Tables 7-18. The A/C Conjugate does not affect immunogenicity to other antigens included in the EPI program.

**Table 7**

| **Descriptive results of Anti-Diphtheria antibodies** **(Seroneutralisation - IU/mL) at V4 -Per protocol analysis** | | | | | | | | |
|---|---|---|---|---|---|---|---|---|
| Anti-Diphtheria (SN - IU/mL) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE | | | Visit V4 | Visit V4 | Visit V4 | Visit V4 | Visit V4 | Visit V4 |
| | N Data (=All-Missing) | | 63 (=63-0) | 64 (=65-1) | 64 (=64-0) | 69 (=69-0) | 81 (=81-0) | 63 (=63-0) |
| | Log10 Dist. {IU/mL} | | | | | | | |
| | | Mean | -0.442 | -0.627 | -0.462 | -0.425 | -0.543 | -0.567 |
| | | Standard Deviation | 0.488 | 0.547 | 0.700 | 0.589 | 0.493 | 0.503 |
| | Distribution {IU/mL} | | | | | | | |
| | | GMT | 0.361 | 0.236 | 0.345 | 0.376 | 0.286 | 0.271 |
| | | [95% CI] | [0.272;0.479] | [0.173;0.324] | [0.231;0.51 6] | [0.271;0.52 1] | [0.223;0.36 8] | [0.203;0.36 3] |
| | | Minimum;Maximum | 0.010;2.56 | 0.020;5.12 | 0.005;10.2 | 0.020;5.12 | 0.020;5.12 | 0.020;2.56 |
| | | Median=Q2 | 0.320 | 0.160 | 0.320 | 0.320 | 0.320 | 0.320 |
| | | Q1;Q3 {Quantiles} | 0.160;0.640 | 0.080;0.640 | 0.160;1.28 | 0.160;1.28 | 0.160;0.640 | 0.160;0.640 |
| | >= 0.01 IU/mL | | | | | | | |
| | | % (n) | 100 (63) | 100 (64) | 98.4 (63) | 100 (69) | 100 (81) | 100 (63) |
| | | [95% CI] | [94.3;100] | [94.4;100] | [91.6;100] | [94.8;100] | [95.5;100] | [94.3;100] |
| | >= 0.1 IU/mL | | | | | | | |
| | | % (n) | 85.7 (54) | 70.3 (45) | 76.6 (49) | 81.2 (56) | 79.0 (64) | 77.8 (49) |
| | | [95% CI] | [74.6;93.3] | [57.6;81.1] | [64.3;86.2] | [69.9;89.6] | [68.5;87.3] | [65.5;87.3] |
| | >= 1 IU/mL | | | | | | | |
| | | % (n) | 22.2 (14) | 12.5 (8) | 29.7 (19) | 29.0 (20) | 11.1 (9) | 15.9 (10) |
| | | [95% CI] | [12.7;34.5] | [5.6;23.2] | [18.9;42.4] | [18.7;41.2] | [5.2;20.0] | [7.9;27.3] |

**Table 8**

| **Descriptive results of Anti-Diphtheria antibodies (Seroneutralisation - IU/mL) at V4 Intent-to-treat analysis** | | | | | | | | |
|---|---|---|---|---|---|---|---|---|
| Anti-Diphtheria (SN - IU/mL) | | | Group#1 | Group#2 | Group#3 | Group#4 | Group#5 | Group#6 |
| | | | (Randomised) | (Randomised) | (Randomise d) | (Randomised ) | (Randomised) | (Randomised) |
| | | | | | | | | |
| BS SCHEDULE | | | Visit V4 | Visit V4 | Visit V4 | Visit V4 | Visit V4 | Visit V4 |
| | N Data (=All-Missing) | | 98 (=104-6) | 96 (=103-7) | 94 (=103-9) | 97 (=103-6) | 101 (=103-2) | 97 (=102-5) |
| | Log10 Dist. {IU/mL} | | | | | | | |
| | | Mean | -0.501 | -0.639 | -0.476 | -0.427 | -0.522 | -0.594 |
| | | Standard Deviation | 0.510 | 0.535 | 0.642 | 0.589 | 0.487 | 0.506 |
| | Distribution {IU/mL} | | | | | | | |
| | | GMT | 0.316 | 0.230 | 0.334 | 0.374 | 0.301 | 0.255 |
| | | [95% CI] | [0.249;0.39 9] | [0.179;0.29 5] | [0.247;0.453 ] | [0.285;0.492] | [0.241;0.375 ] | [0.201;0.322] |
| | | Minimum;Maximum | 0.010;2.56 | 0.020;5.12 | 0.005;10.2 | 0.020;10.2 | 0.020;5.12 | 0.020;2.56 |
| | | Median=Q2 | 0.320 | 0.160 | 0.320 | 0.320 | 0.320 | 0.320 |
| | | Q1;Q3 {Quantiles} | 0.160;0.640 | 0.080;0.640 | 0.160;0.640 | 0.160;1.28 | 0.160;0.640 | 0.160;0.640 |
| | >= 0.01 IU/mL | | | | | | | |
| | | % (n) | 100 (98) | 100 (96) | 98.9 (93) | 100 (97) | 100 (101) | 100 (97) |
| | | [95% CI] | [96.3;100] | [96.2;100] | [94.2;100] | [96.3;100] | [96.4;100] | [96.3;100] |
| | >= 0.1 IU/mL | | | | | | | |
| | | % (n) | 81.6(80) | 69.8 (67) | 77.7 (73) | 82.5 (80) | 80.2 (81) | 76.3 (74) |
| | | [95% CI] | [72.5;88.7] | [59.6;78.7] | [67.9;85.6] | [73.4;89.4] | [71.1;87.5] | [66.6;84.3] |
| | >= 1 IU/mL | | | | | | | |
| | | % (n) | 20.4 (20) | 11.5 (11) | 24.5 (23) | 27.8 (27) | 12.9 (13) | 15.5 (15) |
| | | [95% CI] | [12.9;29.7] | [5.9;19.6] | [16.2;34,4] | [19.2;37.9] | [7.0;21.0] | [8.9;24.2] |

**Table 9**

| **Descriptive results of Anti-Tetanus antibodies (ELISA - IU/mL) at V4 - Per protocol analysis** | | | | | | | | |
|---|---|---|---|---|---|---|---|---|
| Anti-Tetanus (ELISA - IU/mL) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE | | | Visit V4 | Visit V4 | Visit V4 | Visit V4 | Visit V4 | Visit V4 |
| | N Data (=All-Missing) | | 62 (=63-1) | 63 (=65-2) | 64 (=64-0) | 68 (=69-1) | 79 (=81-2) | 63 (=63-0) |
| | Log10 Dist. {IU/mL} | | | | | | | |
| | | Mean | 0.692 | 0.583 | 0.557 | 0.581 | 0.487 | 0.386 |
| | | Standard Deviation | 0.268 | 0.347 | 0.328 | 0.364 | 0.356 | 0.348 |
| | Distribution {IU/mL} | | | | | | | |
| | | GMT | 4.92 | 3.82 | 3.61 | 3.81 | 3.07 | 2.43 |
| | | [95% CI] | [4.21;5.76] | [3.13;4.68 ] | [2.99;4.36] | [3.11;4.66] | [2.56;3.69 ] | [1.99;2.98] |
| | | Minimum;Maximum | 1.00;15.3 | 0.150;17.9 | 0.427;17.9 | 0.075;13.7 | 0.431;20.7 | 0.243;13.8 |
| | | Median=Q2 | 4.97 | 4.31 | 4.13 | 4.46 | 3.28 | 2.74 |
| | | Q1;Q3 {Quantiles} | 3.61;7.56 | 2.14;6.15 | 2.21;6.43 | 2.48;6.60 | 1.84;5.36 | 1.60;4.18 |
| | >= 0.01 IU/mL | | | | | | | |
| | | % (n) | 100 (62) | 100 (63) | 100 (64) | 100 (68) | 100 (79) | 100 (63) |
| | | [95% CI] | [94.2;100] | [94.3;100] | [94.4;100] | [94.7;100] | [95.4;100] | [94.3;100] |
| | >= 0.1 IU/mL | | | | | | | |
| | | % (n) | 100 (62) | 100 (63) | 100 (64) | 98.5 (67) | 100 (79) | 100 (63) |
| | | [95% CI] | [94.2;100] | [94.3;100] | [94.4;100] | [92.1;100] | [95.4;100] | [94.3;100] |
| | >= 1 IU/mL | | | | | | | |
| | | % (n) | 100 (62) | 95.2 (60) | 93.8 (60) | 97.1 (66) | 89.9 (71) | 88.9 (56) |
| | | [95% CI] | [94.2;100] | [86.7;99.0 ] | [84.8;98.3] | [89.8;99.6] | [81.0;95.5 ] | [78.4;95.4] |

**Table 10**

| **Descriptive results of Anti-Tetanus antibodies (ELISA - IU/mL) at V4 - Intent-to-treat analysis** | | | | | | | | |
|---|---|---|---|---|---|---|---|---|
| Anti-Tetanus (ELISA - IU/mL) | | | Group#1 | Group#2 | Group#3 | Group#4 | Group#5 | Group#6 |
| | | | (Randomised ) | (Randomised ) | (Randomised) | (Randomised) | (Randomised) | (Randomised ) |
| | | | | | | | | |
| BS SCHEDULE | | | Visit V4 | Visit V4 | Visit V4 | Visit V4 | Visit V4 | Visit V4 |
| | N Data (=All-Missing) | | 97 (=104-7) | 95 (=103-8) | 92 (=103-11) | 96 (=103-7) | 99 (=103-4) | 97 (=102-5) |
| | Log10 Dist. {IU/mL} | | | | | | | |
| | | Mean | 0.656 | 0.559 | 0.510 | 0.565 | 0.466 | 0.418 |
| | | Standard Deviation | 0.306 | 0.358 | 0.406 | 0.343 | 0.343 | 0.344 |
| | Distribution {IU/mL} | | | | | | | |
| | | GMT | 4.52 | 3.62 | 3.24 | 3.67 | 2.93 | 2.62 |
| | | [95% CI] | [3.93;5.21] | [3.06;4.29] | [2.67;3.93] | [3.13;4.31] | [2.50;3.43] | [2.23;3.07] |
| | | Minimum;Maximu m | 0.596;15.3 | 0.075;17.9 | 0.075;17.9 | 0.075;13.7 | 0.431;20.7 | 0.243;18.1 |
| | | Median=Q2 | 4.73 | 4.10 | 4.00 | 3.90 | 3.02 | 2.82 |
| | | Q1;Q3 {Quantiles} | 2.89;7.47 | 2.58;5.91 | 2.08;6.07 | 2.39;6.47 | 1.81;5.03 | 1.66;4.33 |
| | >= 0.01 IU/mL | | | | | | | |
| | | % (n) | 100 (97) | 100 (95) | 100 (92) | 100 (96) | 100 (99) | 100 (97) |
| | | [95% CI] | [96.3;100] | [96.2;100] | [96.1;100] | [96.2;100] | [96.3;100] | [96.3;100] |
| | >= 0.1 IU/mL | | | | | | | |
| | | % (n) | 100 (97) | 98.9 (94) | 97.8 (90) | 99.0 (95) | 100 (99) | 100 (97) |
| | | [95% CI] | [96.3;100] | [94.3;100] | [92.4;99.7] | [94.3;100] | [96.3;100] | [96.3;100] |
| | >= 1 IU/mL | | | | | | | |
| | | % (n) | 95.9 (93) | 94.7 (90) | 90.2 (83) | 95.8 (92) | 90.9 (90) | 90.7 (88) |
| | | [95% CI] | [89.8;98.9] | [88.1;98.3] | [82.2;95.4] | [89.7;98.9] | [83.4;95.8] | [83.1;95.7] |

**Table 11**

| **Descriptive results of Anti-Poliovirus type 1 antibodies (1/dil.) at V4 - Per protocol analysis** | | | | | | | | |
|---|---|---|---|---|---|---|---|---|
| Anti-Polio 1 (1/dil.) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE | | | Visit V4 | Visit V4 | Visit V4 | Visit V4 | Visit V4 | Visit V4 |
| | N Data (=All-Missing) | | 61 (=63-2) | 62 (=65-3) | 63 (=64-1) | 68 (=69-1) | 79 (=81-2) | 63 (=63-0) |
| | Log10 Dist. {1/dil.} | | | | | | | |
| | | Mean | 1.76 | 1.87 | 1.89 | 2.05 | 1.93 | 1.99 |
| | | Standard Deviation | 0.758 | 0.864 | 0.891 | 0.838 | 0.905 | 0.812 |
| | Distribution {1/dil.} | | | | | | | |
| | | GMT | 57.8 | 74.0 | 78.0 | 112 | 84.8 | 96.7 |
| | | [95% CI] | [37.0;90.4] | [44.7;123] | [46.5;131] | [69.9;178] | [53.1;135] | [60.4;155] |
| | | Minimum;Maximum | 2.00;2048 | 2.00;23170 | 2.00;5793 | 2.00;8192 | 2.00;5793 | 2.00;4096 |
| | | Median=Q2 | 64.0 | 90.5 | 90.5 | 90.5 | 128 | 128 |
| | | Q1;Q3 {Quantiles} | 22.6;181 | 22.6;181 | 22.6;362 | 45.3;512 | 32.0;256 | 22.6;256 |
| | >= 4 1/dil. | | | | | | | |
| | | % (n) | 91.8 (56) | 91.9 (57) | 90.5 (57) | 95.6 (65) | 87.3 (69) | 96.8 (61) |
| | | [95% CI] | [81.9;97.3] | [82.2;97.3] | [80.4;96.4] | [87.6;99.1] | [78.0;93.8] | [89.0;99.6] |

**Table 12**

| **Descriptive results of Anti-Poliovirus type 1 antibodies (1/dil.) at V4 - Intent-to-treat analysis** | | | | | | | | |
|---|---|---|---|---|---|---|---|---|
| Anti-Polio 1 (1/dil.) | | | Group#1 | Group#2 | Group#3 | Group#4 | Group#5 | Group#6 |
| | | | (Randomised) | (Randomised ) | (Randomised) | (Randomised) | (Randomised ) | (Randomised ) |
| | | | | | | | | |
| BS SCHEDULE | | | Visit V4 | Visit V4 | Visit V4 | Visit V4 | Visit V4 | Visit V4 |
| | N Data (=All-Missing) | | 95 (=104-9) | 94 (=103-9) | 91 (=103-12) | 96 (=103-7) | 99 (=103-4) | 96 (=102-6) |
| | Log10 Dist. {1/dil.} | | | | | | | |
| | | Mean | 1.78 | 1.85 | 1.88 | 1.96 | 1.89 | 1.96 |
| | | Standard Deviation | 0.769 | 0.890 | 0.867 | 0.891 | 0.906 | 0.830 |
| | Distribution {1/dil.} | | | | | | | |
| | | | | | | | | |
| | | GMT [95% CI] | 59.9 [41.8;86.0] | 71.2 [46.8;108] | 75.4 [49.8;114] | 91.5 [60.4;139] | 76.8 [50.6;116] | 90.5 [61.5;133] |
| | | Minimum;Max imum | 2.00;5793 | 2.00;23170 | 2.00;5793 | 2.00;8192 | 2.00;5793 | 2.00;8192 |
| | | Median=Q2 | 64.0 | 90.5 | 64.0 | 90.5 | 90.5 | 90.5 |
| | | Q1;Q3 {Quantiles} | 16.0;181 | 22.6;256 | 22.6;256 | 26.9;512 | 22.6;256 | 22.6;256 |
| | >= 4 1/dil. | | | | | | | |
| | | % (n) | 92.6 (88) | 89.4 (84) | 91.2 (83) | 92.7 (89) | 87.9 (87) | 96.9 (93) |
| | | [95% CI] | [85.4;97.0] | [81.3;94.8] | [83.4;96.1] | [85.6;97.0] | [79.8;93.6] | [91.1;99.4] |

**Table 13**

| **Descriptive results of Anti-Poliovirus type 2 antibodies (1/dil.) at V4 - Per protocol analysis** | | | | | | | | |
|---|---|---|---|---|---|---|---|---|
| Anti-Polio 2 (1/dil.) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE | | | Visit V4 | Visit V4 | Visit V4 | Visit V4 | Visit V4 | Visit V4 |
| | N Data (=All-Missing) | | 62 (=63-1) | 63 (=65-2) | 62 (=64-2) | 66 (=69-3) | 79 (=81-2) | 63 (=63-0) |
| | Log10 Dist. {1/dil.} | | | | | | | |
| | | Mean | 2.46 | 2.66 | 2.61 | 2.77 | 2.59 | 2.59 |
| | | Standard Deviation | 0.664 | 0.756 | 0.712 | 0.656 | 0.748 | 0.600 |
| | Distribution {1/dil.} | | | | | | | |
| | | GMT | 286 | 456 | 407 | 584 | 388 | 387 |
| | | [95% CI] | [194;422] | [294;707] | [269;617] | [403;846] | [264;571] | [273;548] |
| | | Minimum;Maximum | 2.00;8192 | 2.00;8192 | 2.00;8192 | 11.3;32768 | 4.00;131072 | 5.70;8192 |
| | | Median=Q2 | 256 | 512 | 609 | 512 | 362 | 362 |
| | | Q1;Q3 {Quantiles} | 128;724 | 181;1448 | 181;1024 | 256;1448 | 181;1024 | 181;1024 |
| | >= 4 1/dil. | | | | | | | |
| | | % (n) | 98.4 (61) | 98.4 (62) | 98.4 (61) | 100 (66) | 100 (79) | 100 (63) |
| | | [95% CI] | [91.3;100] | [91.5;100] | [91.3;100] | [94.6;100] | [95.4;100] | [94.3;100] |

**Table 14**

| **Descriptive results of Anti-Poliovirus type 2 antibodies (1/dil.) at V4 - Intent-to-treat analysis** | | | | | | | | |
|---|---|---|---|---|---|---|---|---|
| Anti-Polio 2 (1/dil.) | | | Group#1 | Group#2 | Group#3 | Group#4 | Group#5 | Group#6 |
| | | | (Randomised) | (Randomised) | (Randomised) | (Randomised) | (Randomised) | (Randomised) |
| | | | | | | | | |
| BS SCHEDULE | | | Visit V4 | Visit V4 | Visit V4 | Visit V4 | Visit V4 | Visit V4 |
| | | N Data (=All-Missing) | 97 (=104-7) | 95 (=103-8) | 90 (=103-13) | 94 (=103-9) 98 | (=103-5) | 97 (=102-5) |
| | Log10 Dist. {1/dil.} | | | | | | | |
| | | Mean | 2.50 | 2.65 | 2.61 | 2.73 | 2.52 | 2.59 |
| | | Standard Deviation | 0.621 | 0.823 | 0.714 | 0.685 | 0.824 | 0.672 |
| | Distribution {1/dil.} | | | | | | | |
| | | GMT | 314 | 446 | 411 | 535 | 333 | 389 |
| | | [95% CI] | [235;419] | [303;656] | [291;580] | [387;739] | [227;487] | [285;531] |
| | | Minimum;Maximu m | 2.00;8192 | 2.00;92682 | 2.00;92682 | 2.00;32768 | 2.00;131072 | 2.00;8192 |
| | | Median=Q2 | 362 | 362 | 512 | 431 | 362 | 362 |
| | | Q1;Q3 {Quantiles} | 128;724 | 181;1448 | 128;1024 | 181;1448 | 128;1024 | 181;1024 |
| | >= 4 1/dil. | | | | | | | |
| | | % (n) | 99.0 (96) | 97.9 (93) | 98.9 (89) | 98.9 (93) | 96.9 (95) | 99.0 (96) |
| | | [95% CI] | [94.4;100] | [92.6;99.7] | [94.0;100] | [94.2;100] | [91.3;99.4] | [94.4;100] |

**Table 15**

| **Descriptive results of Anti-Poliovirus type 3 antibodies (1/dil.) at V4 - Per protocol analysis** | | | | | | | | |
|---|---|---|---|---|---|---|---|---|
| Anti-Polio 3 (1/dil.) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE> | | | Visit V4 | Visit V4 | Visit V4 | Visit V4 | Visit V4 | Visit V4 |
| | N Data (=All-Missing) | | 58 (=63-5) | 61 (=65-4) | 61 (=64-3) | 64 (=69-5) | 75 (=81-6) | 61 (=63-2) |
| | Log10 Dist. {1/dil.} | | | | | | | |
| | | Mean | 2.11 | 2.13 | 1.98 | 2.07 | 2.31 | 2.14 |
| | | Standard Deviation | 0.776 | 0.962 | 0.927 | 0.828 | 0.753 | 0.824 |
| | Distribution {1/dil.} | | | | | | | |
| | | GMT | 130 | 135 | 95.3 | 118 | 205 | 137 |
| | | [95% CI] | [81.0;207] | [76.4;238] | [55.2;165] | [73.3;190] | [138;306] | [84.3;223] |
| | | Minimum;Maximu m | 2.00;2048 | 2.00;23170 | 2.00;4096 | 2.00;5793 | 2.00;4096 | 2.00;4096 |
| | | Median=Q2 | 181 | 181 | 128 | 152 | 256 | 181 |
| | | Q1;Q3 {Quantiles} | 64.0;362 | 32.0;512 | 22.6;362 | 64.0;362 | 90.5;724 | 64.0;512 |
| | >= 4 1/dil. | | | | | | | |
| | | % (n) | 93.1 (54) | 90.2 (55) | 86.9 (53) | 90.6 (58) | 96.0 (72) | 91.8 (56) |
| | | [95% CI] | [83.3;98.1] | [79.8;96.3] | [75.8;94.2] | [80.7;96.5] | [88.8;99.2 ] | [81.9;97.3] |

**Table 16**

| **Descriptive results of Anti-Poliovirus type 3 antibodies (1/dil.) at V4 - Intent-to-treat analysis** | | | | | | | | |
|---|---|---|---|---|---|---|---|---|
| Anti-Polio 3 (1/dil.) | | | Group#1 | Group#2 | Group#3 | Group#4 | Group#5 | Group#6 |
| | | | (Randomised) | (Randomised) | (Randomised) | (Randomised) | (Randomised) | (Randomised) |
| | | | | | | | | |
| BS SCHEDULE | | | Visit V4 | Visit V4 | Visit V4 | Visit V4 | Visit V4 | Visit V4 |
| | N Data (=All-Missing) | | 93 (=104-11) | 92 (=103-11) | 90 (=103-13) | 92 (=103-11) | 95 (=103-8) | 94 (=102-8) |
| | Log10 Dist. {1/dil.} | | | | | | | |
| | | Mean | 2.10 | 2.10 | 2.02 | 2.11 | 2.26 | 2.10 |
| | | Standard Deviation | 0.795 | 0.928 | 0.897 | 0.871 | 0.759 | 0.812 |
| | Distribution {1/dil.} | | | | | | | |
| | | GMT | 125 | 127 | 106 | 128 | 182 | 126 |
| | | [95% CI] | [85.9;183] | [81.6;198] | [68.5;163] | [84.5;194] | [128;260] | [86.0;185] |
| | | Minimum; Maximum | 2.00;5793 | 2.00;23170 | 2.00;4096 | 2.00;5793 | 2.00;4096 | 2.00;4096 |
| | | Median=Q2 | 181 | 181 | 128 | 181 | 181 | 181 |
| | | Q1;Q3 {Quantiles} | 45.3;362 | 45.3;431 | 45.3;362 | 76.1;362 | 90.5;512 | 64.0;362 |
| | >= 4 1/dil. | | | | | | | |
| | | % (n) | 93.5 (87) | 89.1 (82) | 88.9 (80) | 89.1 (82) | 94.7 (90) | 89.4 (84) |
| | | [95% CI] | [86.5;97.6] | [80.9;94.7] | [80.5;94.5] | [80.9;94.7] | [88.1;98.3] | [81.3;94.8] |

**Table 17**

| **Descriptive results of Anti-Agglutinin against Pertussis (1/dil.) at V4 - Per protocol analysis** | | | | | | | | |
|---|---|---|---|---|---|---|---|---|
| Anti-Agglut. Pertussis (1/dil.) | | | Group#1 | Group#2 | Group#3 | Group#4 | Group#5 | Group#6 |
| | | | (Injected) | (Injected) | (Injected) | (Injected) | (Injected) | (Injected) |
| | | | | | | | | |
| BS SCHEDULE | | | Visit V4 | Visit V4 | Visit V4 | Visit V4 | Visit V4 | Visit V4 |
| | N Data (=All-Missing) | | 62 (=63-1) | 63 (=65-2) | 61 (=64-3) | 67 (=69-2) | 77 (=81-4) | 63 (=63-0) |
| | Log10 Dist. {1/dil.} | | | | | | | |
| | | Mean | 2.43 | 2.42 | 2.40 | 2.44 | 2.38 | 2.42 |
| | | Standard Deviation | 0.423 | 0.494 | 0.566 | 0.495 | 0.551 | 0.382 |
| | Distribution {1/dil.} | | | | | | | |
| | | GMT | 271 | 265 | 250 | 275 | 240 | 262 |
| | | [95% CI] | [211;347] | [199;352] | [179;349] | [208;363] | [180;321] | [210;327] |
| | | Minimum;Maximum | 16.0;2048 | 4.00;4096 | 4.00;2048 | 16.0;2048 | 2.00;2048 | 16.0;1024 |
| | | Median=Q2 | 256 | 256 | 256 | 256 | 256 | 256 |
| | | Q1;Q3 {Quantiles} | 128;512 | 128;512 | 128;512 | 128;512 | 128;512 | 128;512 |
| | >= 40 1/dil. | | | | | | | |
| | | % (n) | 93.5 (58) | 93.7 (59) | 91.8 (56) | 91.0 (61) | 90.9 (70) | 98.4 (62) |
| | | [95% CI] | [84.3;98.2] | [84.5;98.2] | [81.9;97.3] | [81.5;96.6] | [82.2;96.3 ] | [91.5;100] |

**Table 18**

| **Descriptive results of Anti-Agglutinin against Pertussis (1/dil.) at V4 - Intent-to-treat analysis** | | | | | | | | |
|---|---|---|---|---|---|---|---|---|
| Anti-Agglut. Pertussis (1/dil.) | | | Group#1 | Group#2 | Group#3 | Group#4 | Group#5 | Group#6 |
| | | | (Randomised) | (Randomised) | (Randomised) | (Randomised) | (Randomised) | (Randomised) |
| | | | | | | | | |
| BS SCHEDULE | | | Visit V4 | Visit V4 | Visit V4 | Visit V4 | Visit V4 | Visit V4 |
| | N Data (=All-Missing) | | 96 (=104-8) | 94 (=103-9) | 90 (=103-13) | 94 (=103-9) | 97 (=103-6) | 97 (=102-5) |
| | Log10 Dist. {1/dil.} | | | | | | | |
| | | Mean | 2.44 | 2.45 | 2.41 | 2.42 | 2.36 | 2.41 |
| | | Standard Deviation | 0.468 | 0.473 | 0.524 | 0.508 | 0.550 | 0.431 |
| | Distribution {1/dil.} | | | | | | | |
| | | GMT | 277 | 282 | 256 | 266 | 232 | 254 |
| | | [95% CI] | [223;345] | [225;352] | [199;330] | [209;338] | [179;299] | [208;310] |
| | | Minimum;Maximum | 16.0;4096 | 4.00;4096 | 4.00;2048 | 2.00;2048 | 2.00;2048 | 16.0;2048 |
| | | Median=Q2 | 256 | 256 | 256 | 256 | 256 | 256 |
| | | Q1;Q3 {Quantiles} | 128;512 | 128;512 | 128;512 | 128;512 | 128;512 | 128;512 |
| | >= 40 1/dil. | | | | | | | |
| | | % (n) | 92.7 (89) | 94.7 (89) | 92.2 (83) | 91.5 (86) | 90.7 (88) | 94.8 (92) |
| | | [95% CI] | [85.6;97.0] | [88.0;98.3] | [84.6;96.8 ] | [83.9;96.3] | [83.1;95.7] | [88.4;98.3] |

For serogroup A, mean SBA titers at 10 months of age did not differ between children who received four A/C Conjugate doses (6, 10, 14 weeks and 9 months) or two doses (14 weeks and 9 months), but are significantly higher than titers of each of the other schedules. For serogroup C, A/C Conjugate at 14 weeks and 9 months induced higher mean SBA titers than did the other regimens. Administration of A/C PS at 24 months led to significantly higher SBA titers in A/C Conjugate recipients, including the two groups receiving single dose conjugate schedules. While responses are lower for serogroup C than A, there is no evidence of hyporesponsiveness.

Meningococcal A/C conjugate vaccine is safe and immunogenic in young infants, particularly when two doses are administered at 14 weeks and 9 months of age. A single dose of A/C Conjugate in the first year of life appears to induce memory.

This study demonstrates that immunogenicity against serogroups A and C is obtained by a number of different administration methods. For example, immunogenicity against serogroups A and C is obtained when children are vaccinated with an A/C conjugate once at 14 weeks of age and a second dose at 9 months of age. Two primary doses of an A/C conjugate given at 6 and 10 weeks of age did not seem to provide any additional benefit. Injection of a single dose, either at 14 weeks or 9 months of age, appeared to provide sufficient long-term protection, based on response to the polysaccharide vaccination at 24 months of age.

A two-dose schedule, whereby the A/C conjugate vaccine is administered at 14 weeks, the time of the DTP3, and again at 9 months, when measles vaccine is given, resulted in immunogenicity against A and C serogroups.

This study demonstrates that A/C conjugate vaccine provides lasting immunologic memory for both serogroup A and C. Borrow R et al., J Infect Dis 2002; 186: 1353-7 have shown comparable results for serogroup C conjugate alone, for infants vaccinated at 2, 3, and 4 months in comparison to those 13-16 months or 4 years of age. Although substantial experience is accumulating in the U.K. for a three dose series of serogroup C meningococcal conjugate vaccine in infants, this study suggests that administration of multiple doses in the first year of life may not be necessary, at least for some conjugate formulations.

This study also demonstrates that administering A/C Conjugate concomitantly with routine infant immunizations such as DTP and OPV, does not interfere with immune response to the other antigens.

### Relevant Paragraphs

1. A method of inducing an immunological response in a patient to capsular polysaccharides A and C of *N. meningitidis* comprising administering an immunologically effective amount of an aluminum-free immunological composition to the patient, wherein the composition comprises two protein-polysaccharide conjugates, the first conjugate comprising a capsular polysaccharide of serogroup A of *N. meningitidis* conjugated to one or more a carrier protein(s) and a second conjugate comprising a capsular polysaccharide of serogroup C of *N. meningitidis* conjugated to one or more a carrier protein(s).
2. The method of Paragraph 1, wherein the carrier protein is a diphtheria toxoid.
3. The method of Paragraph 2, wherein the carrier protein and polysaccharide are covalently attached with a linker.
4. The method of Paragraph 3, wherein the linker is adipic dihydrazide.
5. The method of Paragraph 1, wherein the capsular polysaccharides A and C have an average size of between 5 and 100 kDa.
6. The method of Paragraph 1, wherein the capsular polysaccharides A and C have an average size of between 10 and 75 kDa.
7. The method of Paragraph 1, wherein the capsular polysaccharides A and C have an average size of between 10 and 50 kDa.
8. The method of Paragraph 1, wherein the capsular polysaccharides A and C have an average size of between 10 and 30 kDa.
9. The method of Paragraph 1, wherein the capsular polysaccharides A and C have an average size of between 10 and 25 kDa.
10. The method of Paragraph 1, wherein the composition comprises an adjuvant.
11. The method of Paragraph 1, wherein the immunological composition is administered to the patient in a single dose.
12. The method of Paragraph 11, wherein the patient is less than 12 months of age at the time the immunological composition is administered.
13. The method of Paragraph 1, wherein the immunological composition is administered on the same day or within six months of administration of a vaccine for diphtheria, tetanus, poliovirus, or pertussis.
14. The method of Paragraph 13, wherein the immunological composition is administered on the same day or within three months of administration of a vaccine for diphtheria, tetanus, poliovirus, or pertussis.
15. The method of Paragraph 14, wherein the immunological composition is administered on the same day or within one month of administration of a vaccine for diphtheria, tetanus, poliovirus, or pertussis.
16. The method of Paragraph 15, wherein the immunological composition is administered on the same day of administration of a vaccine for diphtheria, tetanus, poliovirus, or pertussis.
17. The method of Paragraph 14, wherein the vaccine is a poliovirus type 1, 2 or 3.

## Claims

1. An aluminium free composition suitable for use in inducing an immunological response in a patient to capsular polysaccharides A and C of *N. meningitidis,* comprising a capsular polysaccharide of serogroup A of *N. meningitidis* conjugated to one or more carrier protein(s) and a capsular polysaccharide of serogroup C of *N. meningitidis* conjugated to one or more carrier proteins.

2. A composition as claimed in claim 1 wherein the polysaccharides are covalently linked to carrier proteins.

3. A composition as claimed in claim 2 wherein the linker is an adipic dihydrazide derived linker.

4. A composition as claimed in any of claims 1 to 3 wherein the carrier protein is diphtheria toxoid.

5. A composition as claimed in claim 4 for administration in a single dose.

6. A composition as claimed in claim 1 suitable for administration to a patient under twelve months of age at administration.

7. A composition as claimed in any of claims 1 to 6 wherein the capsular polysaccharides have an average molecular weight of 5 to 100 kDa, for example 10 to 75 kDa.

8. A composition as claimed in claim 7 wherein the capsular polysaccharides have an average molecular weight of 10 to 50 kDa, for example 10 to 25 kDa.

9. A composition as claimed in any of claims 1 to 8 for use in inducing an immunological response in a patient to *N. meningitidis* serotypes A and C.

10. A composition for use as claimed in claim 9 wherein the patient is less than twelve months of age.

11. A composition for use as claimed in claim 9 or 10 for administration within six months of administration of a vaccine for diphtheria, tetanus, poliovirus or pertussis.

12. A composition for use as claimed in claim 11 for administration within three months.

13. A composition for use as claimed in claim 11 for administration within one month.

14. A composition for use as claimed in claim 11 for use on the same day.

15. A composition for use as claimed in any of claims 11 to 14 wherein the poliovirus is type 1, 2 or 3.
